# NOUVEAU FASCICULE DE BREVET EUROPEEN

(11) **EP 0 912 484 B2**
(45) Date de publication et mention de la décision concernant l'opposition: **01.06.2005**
(45) Mention de la délivrance du brevet: 11.07.2001
(21) Numéro de dépôt: 97926055.1
(22) Date de dépôt: 29.05.1997
(51) Int. Cl.: C07C 55/14, C07C 51/43

(54) **PROCEDE DE PURIFICATION DE L'ACIDE ADIPIQUE DANS L'EAU**
VERFAHREN ZUR REINIGUNG DER ADIPINSÄURE IM WASSER
METHOD FOR PURIFYING ADIPIC ACID IN WATER

(30) Priorité: 04.06.1996 FR 9607170
(43) Date de publication de la demande: 06.05.1999
(73) Titulaire: Rhodia Polyamide Intermediates, 69192 Saint-Fons (FR)
(72) Inventeur: HENRIET, Eric, B., F-69003 Lyon (FR); LECONTE, Philippe, F-69330 Meyzieu (FR); PATOIS, Carl, F-69003 Lyon (FR); PERRON, Robert, F-69390 Charly (FR)
(74) Mandataire: Esson, Jean-Pierre
(86) Numéro de dépôt international: PCT/FR1997/000938
(87) Numéro de publication internationale: WO 1997/046509

(56) Documents cités:
- EP-A- 0 502 384
- EP-A- 0 712 830
- DE-A- 1 518 242
- DE-A- 1 568 146
- DE-A- 2 624 472
- DE-B- 1 183 489
- DE-C- 737 691
- FR-A- 901 841
- GB-A- 914 510
- GB-A- 1 092 603
- US-A- 3 359 308
- US-A- 3 754 024
- US-A- 3 876 695
- US-A- 4 375 552
- Kirk-Othmer, Encyclopedia of Chemical Technology, 3rd ed., vol. 1, John Wiley & Sons, New York, 510-530 (1978)
- Ullmann's Encyclopedia of Industrial Chemistry, 5th ed., vol. A1, VCH Verlagsgesellschaft mbH, Weiheim (DE), 269-278 (1985)

## Description

La présente invention concerne un procédé de purification de l'acide adipique dans l'eau.

L'acide adipique est l'une des deux matières de base de la préparation du polyamide 6-6. Pour les applications du polyamide 6-6, il est nécessaire d'avoir une très grande pureté et cette pureté doit déjà exister au stade des précurseurs, notamment au stade de l'acide adipique.

Selon le procédé de préparation de l'acide adipique, les impuretés qu'il recèle sont évidemment différentes. En effet, une des impuretés les plus gênantes et parfois les plus coûteuses est constituée par la présence de traces du catalyseur utilisé lors de la préparation de l'acide adipique.

Cependant dans l'exposé qui suit, le procédé sera appliqué à l'acide adipique issu de la double hydroxycarbonylation du butadiène ou encore issu de l'oxydation directe du cyclohexane.

La premiere hydroxycarbonylation du butadiène conduit à un mélanges d'acides penténoïques, principalement à l'acide 3-penténoïque. La deuxième hydroxycarbonylation porte sur les acides penténoïques obtenus dans la première réaction et conduit à l'acide adipique contenant également une certaine quantité d'acide méthyl-2 glutarique, d'acide éthyl-2 succinique, ainsi que d'autres composés, provenant déjà de la première réaction d'hydroxycarbonylation, tels que la gamma-valérolactone, les acides penténoïques non transformés, l'acide méthylbuténoïque. Il contient aussi des traces du catalyseur utilisé dans la deuxième réaction d'hydroxycarbonylation, le plus souvent l'iridium et/ou le rhodium.

L'oxydation directe du cyclohexane en acide adipique est généralement réalisée en présence de cobalt et dans ce procédé l'acide adipique obtenu contient des traces de catalyseur au cobalt.

L'acide adipique étant peu soluble à froid et beaucoup plus soluble à chaud dans l'eau, ce solvant est généralement utilisé pour la cristallisation dudit acide.

Cependant, compte-tenu des puretés très grandes exigées de plus en plus pour l'acide adipique, notamment en ce qui concerne les traces métalliques, une ou même plusieurs recristallisations dans l'eau s'avèrent souvent insuffisantes.

Outre la géne que peut occasionner la présence de traces métalliques pour les diverses utilisations de l'acide adipique, la valeur elle-même de certains catalyseurs comme l'iridium ou le rhodium, compte-tenu des tonnages très importants de l'acide adipique, fait que leur récupération la plus poussée possible est indispensable dans le cadre d'un procédé industriel économiquement viable.

La présente invention consiste en un procédé amélioré de cristallisation ou de recristallisation de l'acide adipique dans l'eau contenant des traces de catalyseur obtenu par hydroxycarbonylation du butadiène ou oxydation directe du cyclohexane, caractérisé en ce que l'acide adipique à purifier a une pureté minimale de 95 % et en ce que ladite cristallisation ou recristallisation est effectuée en présence d'un acide protonique fort et/ou en présence de monoxyde de carbone, la quantité d'acide protonique fort, quand il est présent, est comprise entre 1 et 50 moles par mole de métal de catalyseur contenu dans l'acide adipique à purifier.

Par acide protonique fort, on entend dans le présent texte un acide protonique minéral ayant un pKa inférieur à 1.

A titre d'exemples non limitatifs de tels acides protoniques forts, on peut citer l'acide iodhydrique, l'acide bromhydrique, l'acide chlorhydrique, l'acide nitrique, l'acide sulfurique.

Le monoxyde de carbone peut constituer au moins en partie (de préférence au moins 0,5 bar absolu) l'atmosphère surmontant la solution dans le réacteur de cristallisation ou de recristallisation (ou ciel du réacteur) ou créer dans ledit réacteur une pression supérieure à la pression atmosphérique.

En pratique, on opérera donc sous une pression absolue de 1 à 50 bars de monoxyde de carbone, la limite supérieure n'ayant pas de caractère critique, mais étant représentative d'un appareillage industriel qui ne soit pas excessivement coûteux.

L'acide adipique brut soumis à la recristallisation selon le présent procédé est habituellement un acide adipique ayant déjà subi un ou plusieurs traitements de purification, notamment par cristallisation dans l'eau, par raffinage ou encore par distillation, afin d'avoir une pureté minimale de 95 %.

Généralement l'acide adipique que l'on recristallise par le procédé de l'invention a une pureté de 95 % à 99,95 %.

La recristallisation consiste à dissoudre l'acide adipique à purifier dans le minimum d'eau à chaud, c'est-à-dire habituellement à une température de 80°C à 250°C, en présence d'un acide protonique fort et/ou sous atmosphère ou pression au moins partielle de monoxyde de carbone, puis à faire cristalliser l'acide adipique dissous par un refroidissement de la solution, éventuellement après un ensemencement de la solution à l'aide de cristaux d'acide adipique pur.

Généralement la quantité d'eau utilisée est celle qui conduit à une solution saturée d'acide adipique à la température choisie. A titre indicatif, à 80°C la solution saturée comporte environ 40 % d'acide adipique en poids par poids.

Le procédé de l'invention comprend également la cristallisation de l'acide adipique à partir des mélanges réactionnels qui le contiennent.

C'est ainsi que l'on peut par exemple cristalliser l'acide adipique à partir du mélange obtenu par hydroxycarbonylation d'acide penténoïque par l'eau et le monoxyde de carbone. Ce mélange réactionnel peut être mélangé avec l'eau en présence d'un acide protonique fort et/ou sous atmosphère ou pression au moins partielle de monoxyde de carbone, et l'ensemble est maintenu à une température de 80°C à 250°C, comme indiqué précédemment pour la recristallisation.

Le promoteur utilisé dans la réaction d'hydroxycarbonylation pouvant être l'acide iodhydrique ou l'acide bromhydrique, il peut ne pas être nécessaire de rajouter de l'acide protonique fort. Cependant, on peut si on le souhaite compléter la quantité d'acide protonique fort présente dans le mélange réactionnel.

De même, la réaction d'hydroxycarbonylation étant conduite en présence de monoxyde de carbone, il peut ne pas être nécessaire de rajouter ce composé pour la cristallisation, mais le cas échéant cette possibilité n'est pas exclue. On peut également, comme pour la recristallisation de l'acide adipique, opérer en l'absence de monoxyde de carbone en purgeant l'atmosphère du mélange d'hydroxycarbonylation avant sa cristallisation.

Les exemples qui suivent illustrent l'invention.

### EXEMPLE 1

Dans une ampoule de verre, on charge 5,16 g d'acide adipique contenant 31,0 µg de Co (0,0006 % en poids par poids d'acide adipique) et 7,5 ml d'eau. L'acide adipique a été préparé par oxydation directe du cyclohexane en présence d'acétate de Co et a été purifié par recristallisation dans l'eau. Il ne contient pas d'impuretés organiques en quantités dosables.

L'ampoule ouverte est placée dans un autoclave de 125 ml que l'on ferme.

On met à froid un ciel de monoxyde de carbone (1 bar environ).

On chauffe à 185°C et on maintient environ 30 min à cette température.

Après refroidissement et purge de l'autoclave à l'azote, on filtre l'acide adipique et on rince l'autoclave avec quelques ml d'eau.

On lave l'acide adipique filtré avec 2 fois 5 ml d'eau, puis avec 3 fois 8 ml d'eau.

On sèche l'acide adipique pendant une nuit à l'étuve (60°C). On dose le cobalt présent dans l'acide adipique final par plasma à induction couplée, couplé avec la spectrométrie de masse (IPC/Masse). On trouve 0,000012 % de Co en poids par poids).

### EXEMPLE 2

On répète l'exemple 1 en chargeant 5,21 g du même lot d'acide adipique, contenant 31,2 µg de Co (0,0006 % en poids par poids d'acide adipique), 7,5 ml d'eau et 1 ml d'une solution de 96,7 mg de HCI dans 50 ml d'eau. Le rapport molaire HCI/Co est de 10.

Les conditions opératoires sont les mêmes que pour l'exemple 1, mais le ciel de CO est remplacé par un ciel d'azote (1 bar absolu).

Après lavage et séchage, on dose le cobalt présent dans l'acide adipique final. On trouve 0,00009 % de Co en poids par poids).

### EXEMPLES 3 A 7

On répète l'exemple 1 avec un acide adipique comportant de l'iridium. L'acide adipique a été préparé par hydroxycarbonylation de l'acide 3-penténoïque en présence d'un catalyseur à base d'Ir et a été purifié par recristallisation dans l'eau. Il ne contient pas d'impuretés organiques en quantités dosables.

Le tableau 1 ci-après rassemble les conditions dans lesquelles ont été réalisés les exemples (Tp = température) ainsi que les teneurs initiales et finales en Ir (Ir initial et Ir final) exprimées en microgrammes par gramme de l'acide adipique (AdOH mis en oeuvre.

**Tableau 1**

| **Exemple** | **AdOH en g** | **Eau en g** | **Tp en °C** | **Durée en min** | **CO en bars** | **Rapport molaire HI/Ir** | **Ir initial** | **Ir final** |
|---|---|---|---|---|---|---|---|---|
| Ex 3 | 5,2 | 7,75 | 185 | 30 | 30 | 10 | 2,2 | 0,86 |
| Ex 4 | 5,2 | 9,0 | 185 | 1200 | 1 | 10 | 2,2 | 0,82 |
| Ex 5 | 5,35 | 8,2 | 185 | 1200 | 1 | 20 | 2,2 | 0,92 |
| Ex 6 | 4,9 | 7,25 | 90 | 30 | 0 (1 bar N2) | 10 | 5,0 | 3,8 |
| Ex 7 | 4,95 | 8,0 | 185 | 1200 | 0 (1 bar N2) | 20 | 2,2 | 1,06 |

### EXEMPLE 8

Dans un ballon en verre su rmonté d'un réfrigérant et muni de moyens de chauffage et de refroidissement, on charge 5,44 g d'acide adipique, contenant 0,00095 % de Rh, 7,5 g d'eau et une solution aqueuse de HI (rapport molaire HI/Rh contenu dans l'acide adipique = 10). L'acide adipique a été préparé par hydroxycarbonylation de l'acide 3-penténoïque en présence d'un catalyseur à base de Rh et a été purifié par recristallisation dans l'eau. Il ne contient pas d'impuretés organiques en quantités dosables.

On met à froid un ciel d'azote (1 bar environ).

On chauffe à 90°C et on maintient environ 30 min à cette température.

Après refroidissement, on filtre l'acide adipique et on le lave avec 2 fois 5 ml d'eau saturée en acide adipique.

On sèche l'acide adipique pendant une nuit à l'étuve (60°C). On dose le rhodium présent dans l'acide adipique final. On trouve 0,00054 % de Rh en poids par poids).

### ESSAI COMPARATIF 1

On effectue une recristallisation dans l'eau d'un acide adipique obtenu par hydroxycarbonylation de l'acide 3-penténoïque en présence d'iridium et de Hl. Cet acide adipique a déjà subi une cristallisation et il contient encore 0,00022 % d'iridium.

La recristallisation s'effectue de manière classique par dissolution à 95°C environ de l'acide adipique dans le minimum d'eau, puis par refroidissement progressif de la solution obtenue, puis filtration, enfin lavage de l'acide adipique filtré avec 2 fois 5 ml d'eau et avec 3 fois 8 ml d'eau.

On sèche l'acide adipique pendant une nuit à l'étuve (60°C). On dose l'iridium présent dans l'acide adipique final. On trouve 0,00022 % de Ir en poids par poids). On n'a donc pas réussi à diminuer la teneur en iridium de l'acide adipique.

## Revendications

1. Procédé de purification par cristallisation ou recristallisation dans l'eau de l'acide adipique contenant des traces de catalyseur obtenu par hydroxycarbonylation du butadiène ou oxydation directe du cyclohexane, **caractérisé en ce que** ladite cristallisation ou recristallisation est effectuée à partir d'un acide adipique présentant un degré de pureté d'au moins 95% et en présence d'un acide protonique fort et/ou en présence de monoxyde de carbone, la quantité d'acide protonique fort quand il est présent étant comprise entre 1 et 50 moles par mole de métal catalyseur contenu dans l'acide adipique à purifier.

2. Procédé selon la revendication 1, **caractérisé en ce que** l'acide protonique fort est choisi parmi l'acide iodhydrique, l'acide bromhydrique, l'acide chlorhydrique, l'acide nitrique, l'acide sulfurique.

3. Procédé selon l'une des revendications 1 à 2, **caractérisé en ce que** le monoxyde de carbone constitue au moins en partie l'atmosphère surmontant la solution dans le réacteur de cristallisation ou de recristallisation ou crée dans ledit réacteur une pression supérieure à la pression atmosphérique.

4. Procédé selon l'une des revendications 1 à 3, **caractérisé en ce que** l'on opère sous une pression absolue entre 1 bar et 50 bars de monoxyde de carbone.

5. Procédé selon l'une des revendications 1 à 4, **caractérisé en ce que** l'acide adipique brut soumis à la recristallisation a une pureté de 95 % à 99,95 %.

## Patentansprüche

1. Verfahren zur Reinigung durch Kristallisation oder Umkristallisation in Wasser von Adipinsäure, erhalten durch Hydroxycarbonylierung von Butadien oder durch direkte Oxidation von Cyclohexanon, und enthaltend Katalysatorspuren, **dadurch gekennzeichnet, dass** die besagte Kristallisation oder Umkristallisation, ausgehend von einer Adipinsäure, die einen Reinheitsgrad von mindestens 95% aufweist, und in Gegenwart einer starken Protonensäure und/oder in Gegenwart von Kohlenmonoxid durchgeführt wird, wobei die Menge der starken Protonensäure, sofern anwesend, zwischen 1 und 50 Mol je Mol in der zu reinigenden Adipinsäure enthaltener Metallkatalysator beträgt.

2. Verfahren gemäß Anspruch 1, **dadurch gekennzeichnet, dass** die starke Protonensäure unter Jodwasserstoffsäure, Bromwasserstoffsäure, Chlorwasserstoffsäure, Salpetersäure, Schwefelsäure ausgewählt wird.

3. Verfahren gemäß einem der Ansprüche 1 bis 2, **dadurch gekennzeichnet, dass** das Kohlenmonoxid zumindest einen Teil der über der Lösung in dem Kristallisations- oder Umkristallisationsreaktor liegenden Atmosphäre bildet oder in dem Reaktor einen Druck oberhalb Atmosphärendruck erzeugt.

4. Verfahren gemäß einem der Ansprüche 1 bis 3, **dadurch gekennzeichnet, dass** man unter einem absoluten Druck von 1 bar bis 50 bar Kohlenmonoxid arbeitet.

5. Verfahren gemäß einem der Ansprüche 1 bis 4, **dadurch gekennzeichnet, dass** die der Kristallisation unterzogene rohe Adipinsäure eine Reinheit von 95 bis 99,95% besitzt.

## Claims

1. Process for purifying, by crystallization or recrystallization in water, adipic acid containing catalyst traces obtained by hydroxycarbonylation of butadiene or direct oxidation of cyclohexane, **characterized in that** the said crystallization or recrystallization is carried out from adipic acid having a minimum purity of approximately 95% in the presence of a strong protic acid and/or in the presence of carbon monoxide, the quantity of strong protic acid when it is present, varies from 1 to 50 mol per mole of catalyst metal present in the adipic acid

2. Process according to claim 1, **characterized in that** the strong protic acid is selected from hydroiodic acid, hydrobromic acid, hydrochloric acid, nitric acid and sulphuric acid.

3. Process according to claims 1 or 2, **characterized in that** the carbon monoxide makes up at least part of the atmosphere above the solution in the crystallization or recrystallization reactor or creates within the said reactor a pressure which is greater than the atmospheric pressure.

4. Process according to one of claims 1 to 3, **characterized in that** it is carried out under an absolute pressure of from 1 bar to 50 bars of carbon monoxide.

5. Process according to one of claims 1 to 4, **characterized in that** the crude adipic acid subjected to recrystallization has a purity of from 95 to 99.95%.
